# EUROPÄISCHE PATENTSCHRIFT

(11) **EP 2 924 379 B1**
(45) Veröffentlichungstag und Bekanntmachung des Hinweises auf die Patenterteilung: **19.02.2020**
(21) Anmeldenummer: 15000504.9
(22) Anmeldetag: 23.02.2015
(51) Int. Cl.: F26B 3/347, F26B 15/18, A61L 2/12, A61L 2/18, D06G 1/00

(54) **Verfahren und Vorrichtung zum Behandeln von Matratzen oder dergleichen**
Method and device for the treatment of mattresses or the like
Procédé et dispositif de traitement de matelas ou similaires

(30) Priorität: 20.03.2014 DE 102014003955
(43) Veröffentlichungstag der Anmeldung: 30.09.2015
(73) Patentinhaber: Herbert Kannegiesser GmbH, 32602 Vlotho (DE)
(72) Erfinder: Bringewatt, Wilhelm, 32457 Porta Westfalica (DE); Heinz, Engelbert, 32602 Vlotho (DE)
(74) Vertreter: Möller, Friedrich

(56) Entgegenhaltungen:
- EP-A1- 0 024 304
- EP-A1- 0 634 514
- DE-A1- 2 908 086
- DE-A1- 3 505 571
- DE-C1- 3 729 608

## Beschreibung

Die Erfindung betrifft ein Verfahren zum Behandeln von Matratzen gemäß dem Oberbegriff des Anspruchs 1. Die Erfindung betrifft des Weiteren eine Vorrichtung zum Behandeln von Matratzen gemäß dem Oberbegriff des Anspruchs 9. Die abhängigen Ansprüche beschreiben optionale Ausführungsformen, die auch zur Erfindung gehören. Matratzen oder ähnliche Gegenstände wie beispielsweise Kissen, Polster oder textile Auflagen, müssen, vor allem wenn sie in Hotels oder Krankenhäusern eingesetzt werden, regelmäßig gewaschen und gegebenenfalls desinfiziert werden.

Die Matratzen werden dabei durch die Waschanlage geführt und mit Wasser sowie gegebenenfalls weiteren Behandlungsflüssigkeiten behandelt. Nach dem Waschprozess müssen die nassen Matratzen getrocknet werden. Das geschieht bisher von außen durch einen warmen Luftstrom. Beispielsweise wird in DE 37 29 608 C1 beschrieben, dass eine gewaschene Matratze zum Trockenen mit erwärmter Frischluft beaufschlagt wird.

DE3505571 A1 und DE2908086 A1 offenbaren weitere Verfahren zum Behandeln von Matratzen.

Der Erfindung liegt daher die Aufgabe zugrunde, ein Verfahren und eine Vorrichtung zu schaffen, womit Matratzen auch im Inneren ausreichend getrocknet werden können. Ein Verfahren zur Lösung dieser Aufgabe weist die Maßnahmen des Anspruchs 1 auf. Bei diesem Verfahren ist es vorgesehen, dass die gewaschenen Matratzen durch Bestrahlung mit elektromagnetischen Wellen und/oder Strahlen getrocknet werden. Die elektromagnetischen Wellen erreichen auch das Innere der Matratzen, wodurch sie innerhalb relativ kurzer Zeit vollständig getrocknet werden können. Insbesondere ermöglichen es die elektromagnetischen Wellen bzw. Strahlen, die Matratzen durchgängig bzw. homogen zu trocknen, vorzugsweise kontinuierlich im Durchlauf. Dabei ist es weiter vorgesehen, dass die Matratzen oder durch die Bestrahlung mit elektromagnetischen Wellen durchgängig getrocknet werden, nämlich vom Inneren der Matratze heraus nach. So wird auch der Matratzenkern durch Bestrahlung von den elektromagnetischen Wellen getrocknet. Die Trocknung erfolgt, indem eine Behandlungsflüssigkeit bzw. Wasser im Inneren der Matratze erwärmt und vorzugsweise zum Verdampfen gebracht wird. Die verdampfende Flüssigkeit verlässt die Matratze, was zur wirksamen Trocknung der Matratze führt. Die Wärme wird genau dort deponiert, wo es nass ist. Vorzugsweise lassen sich die elektromagnetischen Wellen derart fokussieren, dass eine größte Energiedichte im Inneren der Matratze erreicht wird. Da der Grad der Erwärmung der Matratze bzw. der Behandlungsflüssigkeit direkt proportional zur Energiedichte der elektromagnetischen Wellen ist, wird der Kern der Matratze zuerst getrocknet. Durch weitere Veränderung der Fokussierung der Wellen kann jeder beliebige Punkt der Matratze mit Bestrahlung beaufschlagt werden.

Insbesondere sieht es die Erfindung weiter vor, dass die Matratzen von mindestens einer ihrer Seiten bzw. Flächen mit elektromagnetischen Wellen und/oder elektromagnetischen Strahlen beaufschlagt werden. Bevorzugt ist es vorgesehen, dass eine Oberseite und eine Unterseite der Matratzen mit den elektromagnetischen Wellen beaufschlagt werden. Dabei können die Seiten bzw. Flächen partiell oder komplett von den elektromagnetischen Wellen erfasst werden. Bei der partiellen Bestrahlung der Matratzen mit elektromagnetischen Wellen werden die Matratzen relativ zu den elektromagnetischen Wellen verfahren, so dass nach und nach die gesamten Matratzen elektromagnetischen Wellen ausgesetzt werden.

Weiter ist es vorgesehen, dass die Matratzen durch eine Zone elektromagnetischer Wellen gefahren werden. Diese Zone kann als ein schmaler Streifen ausgebildet sein oder die gesamte Seite der Matratze einnehmen. Es ist erfindungsgemäß vorgesehen, dass die Matratze liegend durch die Zone der elektromagnetischen Wellen transportiert wird. Alternativ ist es außerdem denkbar, dass die Matratzen stehend bzw. schräg stehend transportiert werden. Insbesondere bei größeren bzw. kleineren Matratzen kann es vorteilhaft sein, wenn diese stehend bzw. schräg stehend transportiert werden.

Es ist weiter erfindungsgemäß vorgesehen, dass die Matratzen beim Trocknen desinfiziert werden. Durch die Bestrahlung der Matratze mit elektromagnetischen Wellen wird die Matratze vom Inneren heraus erhitzt. Das führt dazu, dass die Behandlungsflüssigkeit, insbesondere das Wasser, in der Matratze verdampft. Es wird somit die Energie der elektromagnetischen Strahlung umgewandelt in thermische Energie. Der so erzeugte heiße Wasserdampf tritt durch die Matratze nach außen. Durch diesen heißen Wasserdampf werden jegliche Keime, Bakterien etc. abgetötet.

Insbesondere sieht es die Erfindung weiter vor, dass als elektromagnetische Wellen solche mit einer Wellenlänge von einigen Millimetern bis einigen Metern verwendet werden. Insbesondere sieht es die Erfindung vor, dass die Matratzen mit Mikrowellen bestrahlt werden, die eine Wellenlänge von einigen Millimetern bis einigen Dezimetern, vorzugsweise 1 mm bis 30 cm, aufweisen. Für derartige Wellen ist das Material der Matratze, sowie Wasser transparent. Daher ist es möglich, die Energie der elektromagnetischen Bestrahlung bzw. der Mikrowellen im Inneren der Matratze zu deponieren und ein Verdampfen der Flüssigkeit zu erreichen und dadurch die jeweilige Matratze im Durchlaufverfahren auch im Inneren, vorzugsweise homogen, zu trocknen.

Es ist weiter bevorzugt vorgesehen, dass die Matratzen in einem letzten Waschprozess mit einem Desinfektionsmittel behandelt werden. Dieses Desinfektionsmittel, das beispielsweise in Form einer zusätzlichen Behandlungsflüssigkeit dem Waschprozess zugegeben wird, wird auch beim Trocknen aktiviert und/oder aus der Matratze entfernt. Dadurch, dass die Matratze vom Inneren her getrocknet wird und der entstehende Wasserdampf mit dem Desinfektionsmittel aus dem Inneren der Matratze nach außen wandert, findet eine effektive Desinfektion statt, da sämtliche Bereiche der Matratze von dem heißen Dampf durchdrungen werden.

Es ist des Weiteren bevorzugt vorgesehen, dass die Matratzen vorzugsweise zwischen dem Waschen, insbesondere Spülen bzw. Nachbehandeln, und dem Trocknen entwässert werden. Dieses Entwässern erfolgt durch ein mindestens einmaliges Ausquetschen der Matratzen, so dass ein Teil der gebundenen Flüssigkeit (Flotte) entfernt wird. Bevorzugt erfolgt das Ausquetschen der Matratzen durch mindestens zwei Quetschwalzen, zwischen denen die Matratzen durchgeführt werden. Es ist aber auch denkbar, die Matratzen auf eine andere Art und Weise vor dem Trocknen zu entwässern.

Das erfindungsgemäße Verfahren sieht es weiter vor, dass die Matratzen durch einen gemeinsamen Förderer kontinuierlich durch die mindestens eine Waschzone und Trocknungszone sowie gegebenenfalls eine Spülzone, eine Nachbehandlungszone und/oder eine Entwässerungszone transportiert werden. Die Matratzen werden somit auf dem Förderer kontinuierlich, ohne ihre Positionierung auf dem Förderer zu verändern, durch alle Behandlungszonen transportiert. Dabei wird die Matratze zunächst mit Wasser und/oder einer Behandlungsflüssigkeit beaufschlagt und gewaschen. Dieser Waschprozess kann gegebenenfalls unterstützt werden durch eine mechanische Behandlung, beispielsweise durch Bürsten. In einer Klarwaschzone erfolgt dann ein weiterer Waschprozess. In der Spülzone wird die Matratze von den Behandlungsflüssigkeiten freigespült. In manchen Situationen kann es vorgesehen sein, dass die Matratzen nach dem Spülen noch mit einer weiteren Substanz, beispielsweise einem Desinfektionsmittel, nachbehandelt werden. Nachdem der Waschprozess abgeschlossen ist, werden die nassen Matratzen durch die Entwässerungszone, insbesondere zwei Entwässerungswalzen, befördert, so dass die gebundene Flotte größtenteils entfernt wird. In der folgenden Trocknungszone wird die restliche gebundene Flotte, also die Restfeuchte, durch elektromagnetische Strahlung bzw. Mikrowellenstrahlung entfernt.

Eine Vorrichtung zur Lösung der eingangs genannten Aufgabe weist die Merkmale des Anspruchs 9 auf. Die Vorrichtung zum Behandeln von Matratzen weist mindestens eine Waschzone, eine Trocknungszone und einen Förderer auf, der die Matratzen durch die Wasch- und Trocknungszone transportiert. Erfindungsgemäß weist die Trocknungszone mindestens eine Einrichtung zum Trocknen der Matratzen mit elektromagnetischen Wellen auf, wobei die Einrichtung derart fokussiert ist, dass eine größte Energiedichte im Inneren der Matratze erreicht werden kann. Die Trocknungszone ist derart bemessen, dass sie die Matratzen mindestens teilweise aufnehmen und die elektromagnetischen Wellen bzw. Strahlen auch ins Innere der Matratze gelangen können.

Die erfindungsgemäße Vorrichtung sieht es weiter vor, dass mindestens einer Oberseite der Matratze eine Einrichtung zur Erzeugung von elektromagnetischen Wellen zugeordnet ist. Vorzugsweise ist der Ober- und Unterseite der Matratze oder jeweils mindestens eine Einrichtung zur Erzeugung von elektromagnetischen Wellen zugeordnet. Es ist aber auch denkbar, dass die Einrichtung zur Erzeugung von elektromagnetischen Wellen eine andere relative Anordnung zur Matratze aufweist.

Bevorzugt ist es weiter vorgesehen, dass der Förderer ein Gurtförderer ist, der die Matratzen kontinuierlich durch die einzelnen Behandlungszonen der Vorrichtung, mindestens eine Waschzone und eine Trocknungszone, transportiert. Der Gurtförderer kann einen oder eine beliebige Anzahl von Gurten aufweisen. Als besonders vorteilhaft ist es anzusehen, wenn zwischen den Gurten des Gurtförderers ein Spalt bzw. Freiraum besteht. Durch diesen Spalt wird Wasser oder Behandlungsflüssigkeit, die während des Waschprozesses nicht von den Matratzen aufgenommen wird, abgeführt.

Unter dem Förderer können Wannen zur Aufnahme bzw. zum Sammeln der Flüssigkeiten angeordnet sein. Die Wannen erstrecken sich über den gesamten Förderer oder sind nur unter einigen Zonen, bevorzugt der Wasch-, Spül- und/oder Entwässerungszone, angeordnet. Die gesammelte Behandlungsflüssigkeit kann aufbereitet und dem Waschprozess wieder zugeführt werden.

Der Förderer wird durch Motoren kontinuierlich angertrieben, so dass auch die Matratzen kontinuierlich durch die verschiedenen Zonen der Vorrichtung, mindestens der Wasch- und Trocknungszone, transportiert werden. Die Matratzen werden frei liegend auf dem Förderer transportiert, können aber auch auf dem Förderer fixiert sein.

Weiter ist es bevorzugt vorgesehen, dass es sich bei den elektromagnetischen Strahlen bzw. Wellen um Wellen mit einer Wellenlänge von einigen Millimetern bis einigen Metern handelt, vorzugsweise um Mikrowellen. Die Mikrowellen weisen eine Wellenlänge von einigen Millimetern bis einigen Dezimetern, vorzugsweise 1 mm bis 30 cm, auf. Für Mikrowellen ist das Matratzenmaterial und die Behandlungsflüssigkeit weitgehend transparent. Daher ist es möglich, die Energie der elektromagnetischen Bestrahlung bzw. der Mikrowellen im Inneren der Matratze zu deponieren und ein Verdampfen der Flüssigkeit zu erreichen.

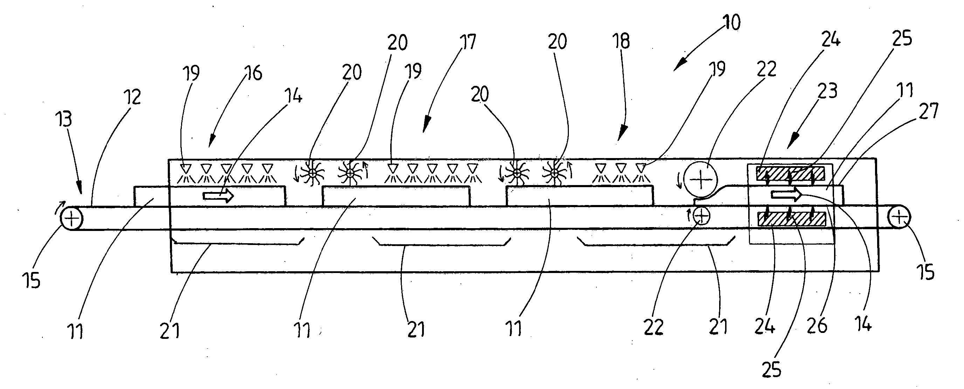

Ein bevorzugtes Ausführungsbeispiel der Erfindung wird nachfolgend anhand der einzigen Figur der Zeichnung näher erläutert.

Die in der Figur dargestellte Vorrichtung 10 dient zum Behandeln, insbesondere mindestens zum Waschen und Trocknen, von Matratzen oder dergleichen. Bei dem in der Figur dargestellten Ausführungsbeispiel werden Matratzen 11 auf einem ein Förderband 12 aufweisenden Förderer 13 in Behandlungsrichtung 14 transportiert. Das Förderband 12 ist endlos um Umlenktrommeln 15 geführt, von denen eine angetrieben wird. Bei dem Förderband 12 kann es sich um ein mindestens einen Gurt aufweisenden Gurtförderer oder auch um einen Rollenförderer handeln. Es ist vorzugsweise vorgesehen, dass das Förderband 12 bzw. die Gurte des Gurtförderers nichtleitend sind, indem sie zum Beispiel aus Gummi oder Kunststoff bestehen.

Die Matratzen 14 werden liegend auf einem Obertrum des Förderbands 12 in Behandlungsrichtung 14 kontinuierlich, vorzugsweise ohne still zu stehen transportiert. Es ist aber auch denkbar, dass die Matratzen 11 auf dem Förderband 12 stehend oder schräg stehend transportiert werden. Dazu können die Matratzen 11 gegebenenfalls auf dem Förderband 12 temporär fixiert sein.

Die zu behandelnde Matratze 12 durchläuft hintereinander, kontinuierlich verschiedene Zonen der gezeigten Vorrichtung 10. Zunächst wird die Matratze 11 einer Vorwaschzone 16 bzw. einem Einweichbereich zugeführt. Auf die Vorwaschzone 16 folgt eine Klarwaschzone 17. Schließlich wird die Matratze 11 in eine Spülzone 18 weitertransportiert.

In der Vorwaschzone 16 sind über der Matratze 11 mehrere Düsen 19 angeordnet. Durch diese Düsen 19 kann die Matratze 11 gleichmäßig mit einer Behandlungsflüssigkeit, zum Beispiel Waser mit oder ohne Waschzusätzen, beaufschlagt werden. Die Vorwaschzone 16 dient dazu, die Matratze 11 mit der Behandlungsflüssigkeit einzuweichen und den Schmutz zu lösen. In der Klarwaschzone 17 wird die Matratze 11 mit einer weiteren Behandlungsflüssigkeit zum Waschen beaufschlagt. Auch hier sind über der Matratze 11 eine Vielzahl von Düsen 19 installiert, die die Matratzen 11 mit einer gegebenenfalls anderen Behandlungsflüssigkeit gleichmäßig besprühen. Es ist auch denkbar, dass Düsen 19 seitlich zu den Matratzen 11 angeordnet sind, um diese auch von der Seite mit Wasser bzw. der Behandlungsflüssigkeit zu beaufschlagen. In der Spülzone 18 wird die jeweilige Matratze 11 aus Düsen 19 von oben, aber auch von den Seiten besprüht. Durch dieses Besprühen der Matratzen 11 mit vorzugsweise Frischwasser werden die Matratzen 11 gespült, so dass Verunreinigungen und Reste der Behandlungsflüssigkeit aus der Matratze 11 entfernt werden. Es ist außerdem denkbar, dass auf die Spülzone 18 eine nicht dargestellte Nachbehandlungszone folgt, in der die Matratze 11 nachbehandelt werden kann. Bei der Nachbehandlung kann es sich beispielsweise um die Beaufschlagung mit einem Desinfektionsmittel und/oder Ausrüstmittel handeln. Ein derartiges Mittel könnte beispielsweise auch mittels Düsen 19 auf die Matratzen 11 aufgetragen werden.

Zwischen der Vorwaschzone 16 und der Klarwaschzone 17 und/oder zwischen der Klarwaschzone 17 und der Spülzone 18 sind Bürstenwalzen 20 vorgesehen. Die Bürstenwalzen 20 drehen sich in Behandlungsrichtung 14 über die Matratzen 11 weg; also in der Darstellung der Figur mit nach rechts verlaufender Behandlungsrichtung 14 entgegen dem Uhrzeigersinn. Die Bürstenwalzen 20 erstrecken sich über die gesamte Breite der Matratze 11 bzw. über die gesamte Behandlungsfläche. Die Drehachsen der Bürstenwalzen 20 verlaufen quer zur Behandlungsrichtung 14 der Matratzen 11 mit parallelem Abstand über dem Obertrum des Förderers 13. Durch das mechanische Einwirken der Bürsten auf die Oberfläche der Matratzen 11 wird die Behandlungsflüssigkeit bzw. das Wasser effektiv in die Matratze 11 eingearbeitet. Außerdem ist es vorgesehen, dass die Bürsten die Oberfläche der Matratzen 11 leicht aufrauen und so die Aufnahme der Behandlungsflüssigkeit verbessert wird. Die Anzahl der Bürstenwalzen 20 und die Positionierung derselben ist nicht auf die in der Figur dargestellte Weise festgelegt.

Es ist weiter vorgesehen, dass die Vorrichtung 10 nicht dargestellte Press- oder Walkwalzen aufweist. Diese Walkwalzen sind quer zur Behandlungsrichtung 14 über bzw. unter den Matratzen 11 angeordnet und dienen dem Durchwalken bzw. dem Zusammenstauchen der Matratzen 11. So können die Matratzen 11 beispielsweise zwischen zwei Walkwalzen zusammengestaucht werden, oder zwischen dem Förderband 12 und einer Walkwalze. Durch dieses Stauchen und Wiederaufweiten der Matratzen saugen diese besonders effektiv Waser und Behandlungsflüssigkeit auf, wodurch der gesamte Matratzenkörper, auch im Kern, durchtränkt und die Behandlungsflüssigkeit durch den Matratzenkörper getrieben wird. Dieses Durchwalken kann periodisch wiederholt werden.

Unter der Waschzone 16 bzw. unter der Klarwaschzone 17 und der Spülzone 18 sind jeweils eine Wanne 21 vorgesehen, welche Wasser und Behandlungsflüssigkeit, die nicht von der Matratze 11 aufgenommen werden, sammeln bzw. auffangen. Die von den Wannen 21 aufgenommene bzw. gesammelte Behandlungsflüssigkeit bzw. Wasser kann den Waschprozessen entweder wieder zugeführt oder wieder aufbereitet werden. Die Wannen 21 sind nicht auf die dargestellte Ausführungsform der Figur beschränkt. Vielmehr können sich die Wannen 21 über den gesamten Wasch- und Spülbereich der Matratzen 11 durchgehend erstrecken.

Am Ende der Spülzone 18 folgen in Behandlungsrichtung 14 zwei gegenüberliegende Quetschwalzen 22. Die Quetschwalzen 22 sind quer zur Behandlungsrichtung 14 ausgerichtet und rotieren gegensinnig. Der Abstand der beiden Quetschwalzen 22 ist kleiner als die Dicke der Matratze 11. Die gewaschene Matratze 11, welche noch die gebundene Flotte aufweist, wird von dem Förderband 12 durch die beiden Quetschwalzen 22 geführt. Durch das Zusammenquetschen der Matratze 11 wird die Flotte mindestens teilweise aus der Matratze 11 herausgepresst. Die herausgepresste Flotte wird ebenfalls von einer Wanne 21 aufgefangen.

Nachdem die Matratze 11 von den Quetschwalzen 22 zumindest von einem Teil der gebundenen Flotte befreit wurde, wird die Matratze 11 durch den Förderer 13 einer Trocknungseinrichtung 23 der Vorrichtung 10 zugeführt. Die in der Figur dargestellte Trocknungseinrichtung 23 der Matratze 11 weist zwei gegenüberliegende Sender 24 zur Erzeugung von Mikrowellenstrahlung auf. Die Trocknungseinrichtung 23 ist jedoch nicht auf das Spektrum der Mikrowellen eingeschränkt, sondern kann vielmehr auch Strahlung erzeugen, die über dieses Spektrum hinausgeht. Die in der Figur dargestellten Sender 24 sind parallel zu einer Oberseite 27 und einer Unterseite 26 der Matratze 11 angeordnet. Die Sender 24 erzeugen flächig Mikrowellenstrahlung, die in Richtung Matratze 11 gerichtet sind. Da Textilien und Wasser transparent sind für Mikrowellenstrahlung, dringen die Mikrowellen 25 in den Körper bzw. Kern der Matratze 11 ein. Durch entsprechende Ausrichtung der Sender 24 bzw. durch Fokussierung der Mikrowellen 25 kann im Inneren der Matratze 11 eine Energiedichte erreicht werden, die zum Erhitzen des Wassers bzw. der Behandlungsflüssigkeit ausreicht. Durch Erhitzen der Behandlungsflüssigkeit bzw. des Wassers verdampft dieses und tritt durch die Matratze 11 nach außen. Auf diese Weise wird die Matratze 11 im Durchlaufverfahren vollständig, auch im inneren Kern, getrocknet. Diese Trocknung erfolgt durchgehend über den gesamten Querschnitt der Matratze 11, und zwar im Wesentlichen homogen. Dabei durchströmt der heiße Dampf die Matratze 11 und tötet sämtliche Keime und Bakterien ab. Falls die Matratze 11 vor der Trocknung noch mit einem Desinfektionsmittel beaufschlagt wurde, wird dies ebenfalls direkt verdampft und durch den heißen Dampf aus dem Matratzenkörper hinausgetragen. Dadurch wird eine effiziente Desinfektion der Matratze 11 von sämtlichen Keimen und Bakterien aus dem Inneren heraus erreicht.

Da die Matratze 11 auf dem Förderband 12 kontinuierlich durch die Trocknungseinrichtung 23 bzw. zwischen den Sendern 24 hindurchtransportiert wird, kann der effektive Bereich, der mit Mikrowellen 25 beaufschlagt wird, kleiner als die Gesamtfläche der Unterseite 26 bzw. Oberseite 27 der Matratze 11 sein. Je nach Transportgeschwindigkeit der Matratze 11 auf dem Förderband 12 und somit je nach Verweildauer der Matratze 11 zwischen den Sendern 24 kann die Strahlungsleistung der Mikrowellen 25 angepasst werden. So führt bei einer langsamen Transportgeschwindigkeit der Matratze 11 und einer geringen Leistung der Sender 24 zu einer gleichen Wirkung wie eine schnelle Transportgeschwindigkeit der Matratze 11 und eine hohe Sendeleistung der Sender 24. Dadurch, dass die Mikrowellen 25 von den Sender 24 in verschiedenen Ebenen der Matratze 11 fokussiert werden können, lässt sich jeder Bereich im Inneren bzw. im Kern der Matratze 11 erreichen bzw. aufheizen.

### Bezugszeichenliste:

- 10: Vorrichtung
- 11: Matratze
- 12: Förderband
- 13: Förderer
- 14: Behandlungsrichtung
- 15: Umlenktrommel
- 16: Vorwaschzone
- 17: Klarwaschzone
- 18: Spülzone
- 19: Düse
- 20: Bürstenwalze
- 21: Wanne
- 22: Quetschwalze
- 23: Trocknungseinrichtung
- 24: Sender
- 25: Mikrowelle
- 26: Unterseite
- 27: Oberseite

## Patentansprüche

1. Verfahren zum Behandeln von Matratzen (11), wobei die Matratzen (11) gewaschen, entwässert und getrocknet werden, wobei das Entwässern durch ein Ausquetschen der Matratzen (11) erfolgt, **dadurch gekennzeichnet, dass** die Matratzen (11) durch Bestrahlung mit elektromagnetischen Wellen getrocknet werden, wobei die elektromagnetischen Wellen derart fokussiert werden, dass eine größte Energiedichte im Inneren der Matratze (11) erreicht wird, so dass die Matratze (11) von innen nach außen getrocknet werden.

2. Verfahren nach Anspruch 1, **dadurch gekennzeichnet, dass** die Matratzen (11) von mindestens einer ihrer Seiten, vorzugsweise einer Oberseite (27) und einer Unterseite (26), mit elektromagnetischen Wellen beaufschlagt werden.

3. Verfahren nach Anspruch 1 oder 2, **dadurch gekennzeichnet, dass** die Matratzen (11) durch eine Zone elektromagnetische Wellen gefahren werden, vorzugsweise flach liegend.

4. Verfahren nach einem der vorhergehenden Ansprüche, **dadurch gekennzeichnet, dass** die Matratzen (11) beim Trocknen desinfiziert werden, insbesondere durch von den elektromagnetischen Wellen erzeugten thermischen Energie.

5. Verfahren nach einem der vorhergehenden Ansprüche, **dadurch gekennzeichnet, dass** als elektromagnetische Wellen solche mit einer Wellenlänge von einigen Millimetern bis einigen Metern verwendet werden, vorzugsweise Mikrowellen (25).

6. Verfahren nach einem der vorhergehenden Ansprüche, **dadurch gekennzeichnet, dass** die Matratzen (11) in einem letzten Waschprozess mit einem Desinfektionsmittel behandelt werden.

7. Verfahren nach einem der vorhergehenden Ansprüche, **dadurch gekennzeichnet, dass** die Matratzen (11) zwischen dem Spülen bzw. Nachbehandeln und dem Trocknen entwässert werden, vorzugsweise die freie Flotte aus den Matratzen (11) ausgequetscht wird.

8. Verfahren nach einem der vorhergehenden Ansprüche, **dadurch gekennzeichnet, dass** die Matratzen (11) durch einen gemeinsamen Förderer (13) kontinuierlich durch die mindestens eine Waschzone (16, 17), eine Spülzone (18), gegebenenfalls eine Nachbehandlungszone, eine Entwässerungszone und eine Trocknungszone transportiert werden.

9. Vorrichtung (10) zum Behandeln von Matratzen (11) mit mindestens einer Waschzone (16, 17), einer Entwässerungszone und einer Trocknungszone und mit einem Förderer (13) der die Matratzen (11) durch die Waschzone (16, 17), die Entwässerungszone und die Trocknungszone transportiert, wobei die Entwässerungszone zwei Quetschwalzen (22) zum Ausquetschen der Matratzen (11) aufweist, **dadurch gekennzeichnet, dass** die Trocknungszone mindestens eine Einrichtung (23) zum Trocknen der Matratzen (11) mit elektromagnetischen Wellen aufweist, wobei die Einrichtung (23) derart fokussierbar ist, dass eine größte Energiedichte im Inneren der Matratze (11) erreicht werden kann.

10. Vorrichtung (10) nach Anspruch 9, **dadurch gekennzeichnet, dass** mindestens einer Oberseite (27) der Matratzen (11) eine Einrichtung (23) zur Erzeugung von elektromagnetischen Wellen zugeordnet ist, vorzugsweise der Ober- und Unterseite (26, 27) der Matratze (11) jeweils mindestens eine Einrichtung (23) zur Erzeugung von elektromagnetischen Wellen zugeordnet ist.

11. Vorrichtung (10) nach einem der Ansprüche 9 bis 10, **dadurch gekennzeichnet, dass** der Förderer (13) ein Gurtförderer ist, der die Matratzen (11) kontinuierlich durch mindestens eine Waschzone (16, 17) und/oder eine Spülzone (18), gegebenenfalls eine Nachbehandlungszone, eine Entwässerungszone und eine Trocknungszone transportiert.

12. Vorrichtung (10) nach einem der Ansprüche 9 bis 11, **dadurch gekennzeichnet, dass** dem Förderer (13) mindestens eine Wanne (21) zugeordnet ist zum Sammeln und Auffangen einer Behandlungsflüssigkeit.

13. Vorrichtung nach einem der Ansprüche 9 bis 12, **dadurch gekennzeichnet, dass** es sich bei den elektromagnetischen Wellen um Wellen mit einer Wellenlänge von einigen Millimetern bis einigen Metern handelt, vorzugsweise um Mikrowellen (25).

## Claims

1. Method for treating mattresses (11), the mattresses being washed, dewatered and dried, the dewatering being performed by squeezing out the mattresses (11), **characterized in that** the mattresses (11) are dried by irradiation with electromagnetic waves, the electromagnetic waves being focused in such a way that a greatest energy density is achieved in the interior of the mattress (11), so that the mattress (11) is dried from the inside outwards.

2. Method according to Claim 1, **characterized in that** the mattresses (11) are subjected to electromagnetic waves from at least one of their sides, preferably an upper side (27) and an underside (26).

3. Method according to Claim 1 or 2, **characterized in that** the mattresses (11) are passed through a zone of electromagnetic waves, preferably while lying flat.

4. Method according to one of the preceding claims, **characterized in that** the mattresses (11) are disinfected during drying, in particular by thermal energy generated by the electromagnetic waves.

5. Method according to one of the preceding claims, **characterized in that** waves with a wavelength of several millimetres to several metres, preferably microwaves (25), are used as the electromagnetic waves.

6. Method according to one of the preceding claims, **characterized in that** in a last washing process the mattresses (11) are treated with a disinfectant.

7. Method according to one of the preceding claims, **characterized in that**, between the rinsing or aftertreatment and the drying, the mattresses (11) are dewatered, preferably the free wash liquor is squeezed out of the mattresses (11).

8. Method according to one of the preceding claims, **characterized in that** the mattresses (11) are continuously transported through the at least one washing zone (16, 17), a rinsing zone (18), possibly an aftertreatment zone, a dewatering zone and a drying zone by a common conveyor (13).

9. Apparatus (10) for treating mattresses (11), comprising at least one washing zone (16, 17), a dewatering zone and a drying zone and comprising a conveyor (13), which transports the mattresses (11) through the washing zone (16, 17), the dewatering zone and the drying zone, the dewatering zone having two squeezing rolls (22) for squeezing out the mattresses (11), **characterized in that** the drying zone has at least one device (23) for drying the mattresses (11) with electromagnetic waves, the device (23) being focusable in such a way that the greatest energy density can be achieved in the interior of the mattress (11).

10. Apparatus (10) according to Claim 9, **characterized in that** at least an upper side (27) of the mattresses (11) is assigned a device (23) for generating electromagnetic waves, preferably the upper side (26) and the underside (27) of the mattress (11) are respectively assigned at least one device (23) for generating electromagnetic waves.

11. Apparatus (10) according to either of Claims 9 and 10, **characterized in that** the conveyor (13) is a belt conveyor, which continuously transports the mattresses (11) through at least one washing zone (16, 17) and/or a rinsing zone (18), possibly an aftertreatment zone, a dewatering zone and a drying zone.

12. Apparatus (10) according to one of Claims 9 to 11, **characterized in that** the conveyor (13) is assigned at least one tray (21) for collecting and catching a treatment liquid.

13. Apparatus according to one of Claims 9 to 12, **characterized in that** the electromagnetic waves are waves with a wavelength of several millimetres to several metres, preferably microwaves (25).

## Revendications

1. Procédé de traitement de matelas (11), les matelas (11) étant lavés, essorés et séchés, l'essorage étant effectué par compression des matelas (11), **caractérisé en ce que** les matelas (11) sont séchés par irradiation avec des ondes électromagnétiques, les ondes électromagnétiques étant focalisées de manière à obtenir une plus grande densité d'énergie à l'intérieur du matelas (11) de façon à sécher les matelas (11) de l'intérieur vers l'extérieur.

2. Procédé selon la revendication 1, **caractérisé en ce que** les matelas (11) sont soumis à des ondes électromagnétiques depuis au moins un de leurs côtés, de préférence un côté supérieur (27) et un côté inférieur (26).

3. Procédé selon la revendication 1 ou 2, **caractérisé en ce que** les matelas (11) sont déplacés à travers une zone d'ondes électromagnétiques, de préférence à plat.

4. Procédé selon l'une des revendications précédentes, **caractérisé en ce que** les matelas (11) sont désinfectés lors du séchage, notamment par l'énergie thermique générée par les ondes électromagnétiques.

5. Procédé selon l'une des revendications précédentes, **caractérisé en ce que** les ondes électromagnétiques utilisées ont une longueur d'onde de quelques millimètres à quelques mètres, de préférence des micro-ondes (25).

6. Procédé selon l'une des revendications précédentes, **caractérisé en ce que** les matelas (11) sont traités avec un désinfectant dans un dernier processus de lavage.

7. Procédé selon l'une des revendications précédentes, **caractérisé en ce que** les matelas (11) sont essorés entre le rinçage ou le post-traitement et le séchage, de préférence le bain libre étant pressuré des matelas (11).

8. Procédé selon l'une des revendications précédentes, **caractérisé en ce que** les matelas (11) sont transportés par un convoyeur commun (13) en continu à travers l'au moins une zone de lavage (16, 17), une zone de rinçage (18), éventuellement une zone de post-traitement, une zone d'essorage et une zone de séchage,

9. Dispositif (10) de traitement de matelas (11) comprenant au moins une zone de lavage (16, 17), une zone d'essorage et une zone de séchage ainsi qu'un convoyeur (13) qui transporte les matelas (11) à travers la zone de lavage (16, 17), la zone d'essorage et la zone de séchage, la zone d'essorage comportant deux rouleaux presseurs (22) destinés à pressurer les matelas (11), **caractérisé en ce que** la zone de séchage comporte au moins un moyen (23) destiné à sécher les matelas (11) avec des ondes électromagnétiques, le moyen (23) pouvant être focalisé de manière à obtenir une plus grande densité d'énergie à l'intérieur du matelas (11).

10. Dispositif (10) selon la revendication 9, **caractérisé en ce qu'**au moins un côté supérieur (27) des matelas (11) est associé à un moyen (23) destiné à générer des ondes électromagnétiques, de préférence le côté supérieur et le côté inférieur (26, 27) des matelas (11) étant associés à au moins un moyen (23) destiné à générer des ondes électromagnétiques.

11. Dispositif (10) selon l'une des revendications 9 et 10, **caractérisé en ce que** le convoyeur (13) est un convoyeur à bande qui transporte les matelas (11) en continu à travers au moins une zone de lavage (16, 17) et/ou une zone de rinçage (18), éventuellement une zone de post-traitement, une zone d'essorage et une zone de séchage.

12. Dispositif (10) selon l'une des revendications 9 à 11, **caractérisé en ce que** le convoyeur (13) est associé à au moins un bac (21) destiné à collecter et recueillir un liquide de traitement.

13. Dispositif selon l'une des revendications 9 à 12, **caractérisé en ce que** les ondes électromagnétiques sont des ondes ayant une longueur d'onde de quelques millimètres à quelques mètres, de préférence des micro-ondes (25).
